# FASCICULE DE BREVET EUROPEEN

(11) **EP 3 552 012 B1**
(45) Date de publication et mention de la délivrance du brevet: **06.11.2024**
(21) Numéro de dépôt: 17808484.4
(22) Date de dépôt: 05.12.2017
(51) Int. Cl.: G01N 33/28, G01N 33/18, G01N 33/00

(54) **PROCEDE DE CONTROLE DE LA CONCENTRATION DE COMPOSES ORGANIQUES VOLATILS DANS UN FLUIDE DE FORAGE**
VERFAHREN ZUR STEUERUNG DER KONZENTRATION VON FLÜCHTIGEN ORGANISCHEN VERBINDUNGEN IN EINER BOHRFLÜSSIGKEIT
METHOD FOR CONTROLLING THE CONCENTRATION OF VOLATILE ORGANIC COMPOUNDS IN A DRILLING FLUID

(30) Priorité: 08.12.2016 FR 1662143
(43) Date de publication de la demande: 16.10.2019
(73) Titulaire: Suez International, 92040 Paris La Défense Cedex (FR)
(72) Inventeur: BRUCHET, Auguste, 78400 Chatou (FR); DO QUANG, Zdravka, 78870 Bailly (FR); BAUDIN, Isabelle, 92000 Nanterre (FR); NOYON, Naike, 91430 Igny (FR)
(74) Mandataire: Lavoix
(86) Numéro de dépôt international: PCT/EP2017/081444
(87) Numéro de publication internationale: WO 2018/104262

(56) Documents cités:
- WO-A1-2009/140114
- CN-A- 102 735 859
- US-A- 5 435 169
- US-A1- 2010 292 844
- US-A1- 2013 029 427
- US-A1- 2016 102 510
- WORTBERG M ET AL: "Monitoring industrial wastewater by online GC-MS with direct aqueous injection", ANALYTICAL AND BIOANALYTICAL CHEMISTRY, SPRINGER, BERLIN, DE, vol. 384, no. 5, 1 March 2006 (2006-03-01), pages 1113 - 1122, XP019327885, ISSN: 1618-2650, DOI: 10.1007/S00216-005-3366-2

## Description

L'invention se situe dans le domaine de la mesure des composés organiques en ligne dans un réseau de fluide et concerne un procédé de contrôle de la concentration de composés organiques dans un fluide d'un réseau de fluide. L'invention concerne aussi un dispositif de contrôle de la concentration de composés organiques dans un fluide d'un réseau de fluide.

Les eaux potables sont définies par quelques dizaines de paramètres non spécifiques (température, pH, conductivité, turbidité...), microbiologiques (coliformes, germes totaux...) et chimiques organiques (pesticides, hydrocarbures, solvants chlorés, trihalométhanes...) et inorganiques (métaux, bromates...). Le contrôle de la conformité est réalisé au moyen de prélèvements d'échantillons et d'analyses au laboratoire dont les résultats sont connus dans un délai allant de douze heures à quelques semaines. Par ailleurs, la fréquence de ces analyses peut varier dans le meilleur des cas de journalière (microbiologie sur les grandes installations) à une par mois pour les paramètres chimiques organiques et inorganiques, ceci pour les grosses installations. Pour des installations délivrant moins de 1000 m3/jour, cette fréquence peut n'être qu'annuelle. Il est donc clair que dans les intervalles entre ces analyses de laboratoires, des non conformités peuvent survenir sans que l'on ne les détecte, donc sans possibilité d'action au niveau des installations de traitement. Une solution idéale consisterait à piloter les installations de traitement au moyen des informations fournies de façon quotidienne ou plusieurs fois par jour, par des systèmes de mesure en continu ou semi-continu, en amont et/ou aval des traitements.

Il existe déjà des sondes de mesure en continu pour un nombre limité de paramètres physico-chimiques simples tels que pH, turbidité, conductivité, température, absorbance UV, mais aucune installation de traitement permettant de contrôler la concentration de composés organiques n'a été à ce jour identifiée.

Il apparait donc nécessaire d'avoir une installation de traitement pilotée ou optimisée par des informations provenant d'analyseurs en ligne de composés organiques spécifiques (individuels) en particulier de composés organiques volatils (COV). Les COV étant définis en France (décret n°2006-623) comme tout composé organique dont le point d'ébullition, mesuré à la pression standard de 101,3 kPa, est inférieur ou égal à 250°C (cf. tableau ci-dessous). Les COV sont à considérer du fait de leur toxicité ou génération de goûts et odeurs. Les composés notamment pertinents pour des applications en eau potable sont ceux les plus occurents au niveau des ressources, dont certains sont réglementés (en gras dans le tableau ci-dessous ) : le chlorure de vinyle, le 1,1 dichloroéthane, l'ETBE, le 1,1,1 trichloroéthane, le chloroforme, le 1,2 cis dichloroéthylène, le trichloroéthylène et le tetrachloroéthylène :

| **Famille** | **Composé** | **Pression de vapeur saturante (Pa à 20°C)** | **Température d'ébullition** |
|---|---|---|---|
| Hydrocarbures halogénés (COHV) | dichlorométhane | 4,65.10⁴ | 40°C |
| | trichloroéthylène | 8,6.10³ | 87°C |
| | tetrachloroéthylène | 1,9.10³ | 121°C |
| Hydrocarbures aromatiques (BTEX) | benzène | 9,97.10³ | 80,1°C |
| | toluène | 3,9.10³ | 110,58°C |
| | o-xylène | 663 | 144,43°C |
| | éthylbenzène | 900 | 136,2°C |
| Ethers | méthyl tert butyl éther (MTBE) | 2,7.10⁴ | 55,05°C |
| | éthyl tert butyl éther (ETBE) | 1,7.10⁴ | 73°C |

| **Paramètre** | **OMS (2011)** | **France (directive 98/83/CE)** | **Australie** | E**tats-Unis (US EPA)** |
|---|---|---|---|---|
| 1,1 dichloroéthylène | | | | 7 µg/L |
| 1,2 dichloroéthylène | 50 µg/L | | | |
| 1,2 cis dichloroéthylène | | | | 70 µg/L |
| 1,2 trans dichloroéthylène | | | | 100 µg/L |
| 1,2 dichloroéthane | 30 µg/L | 3 µg/L | 3 µg/L | 5 µg/L |
| 1,2 dichlorobenzène | 1 000 µg/L | | | |
| 1,1,1 trichloroéthane | | | | 200 µg/L |
| 1,2,4 trichlorobenzène | | | | 70 µg/L |
| Benzène | 10 µg/L | 1 µg/L | 1 µg/L | 5 µg/L |
| Chlorobenzènes | | | | 100 µg/L |
| **Chloroforme** | **300 µg/L** | **ΣTHMs** = **100 µg/L** | | **80 µg/L** |
| **Chlorure de vinyle** | **0,3 µg/L** | **0,5 µg/L** | **0,3 µg/L** | **2** µ**g/L** |
| Dichlorométhane | 20 µg/L | | | 5 µg/L |
| Ethylbenzène | 300 µg/L | | | 700 µg/L |
| Styrène | 20 µg/L | | | 100 µg/L |
| Tetrachlorure de carbone | 4 µg/L | | | 5 µg/L |
| **Tetrachloroéthylène** | **40 µg/L** | **Σ = 10 µg/L** | | **5 µg/L** |
| **Trichloroéthylène** | **20 µg/L** | | | **5 µg/L** |
| Toluène | 700 µg/L | | | 1 000 µg/L |
| Xylènes (total) | 500 µg/L | | | 10 µg/L |

Mais il existe un préjugé lié à la complexité des chromatographes de laboratoire et à l'expertise requise pour les utiliser.

Le document US2016/102510 A1 décrit la mesure des gaz dangereux ou toxiques dans un fluide de forage et la diminution d'une concentration excessive en ajoutant des additifs ou de l'eau.

L'invention vise à pallier tout ou partie des problèmes cités plus haut en proposant un procédé de contrôle de la concentration de composés organiques dans un fluide d'un réseau de fluide en faisant une mesure en ligne des composés organiques et dont le résultat des mesures permet de piloter un procédé industriel de façon à diminuer la concentration de composés organiques si celle-ci est trop élevée. Ce procédé permet d'améliorer l'utilisation opérationnelle des données issues du suivi des composés organiques pour les réseaux d'eau. Il permet de garantir des performances identiques à celles des méthodes de référence de laboratoire (avec un écart maximal admissible assez faible) et donc d'optimiser les paramètres opérationnels de la filière de traitement sans risque de surdosage ou de sous-dosage de produits destinés à diminuer la concentration des composés organiques.

A cet effet, l'invention a pour objet un procédé de contrôle de la concentration de composés organiques dans un fluide d'un réseau de fluide d'une installation industrielle selon la revendication 1.

Selon un mode de réalisation hors du cadre de l'invention, la troisième étape de diminution comprend une étape d'injection dans le réseau de fluide d'unt produit ayant pour effet de diminuer la concentration du au moins un composé organique. Il peut s'agir d'une étape d'injection dans le réseau de fluide d'un réactif (tel qu'un media adsorbant, un oxydant, un gaz de stripping) ou mise en oeuvre d'un procédé de traitement (tel que filtration membranaire).

Selon un autre mode de réalisation, le procédé de contrôle selon l'invention comprend préalablement à la troisième étape de diminution une étape de télétransmission de la concentration de chacun des composés organiques de la pluralité de composés organiques mesurée par l'analyseur à un centre de contrôle.

Selon un mode de réalisation hors du cadre de l'invention, le procédé industriel est un procédé de traitement d'un fluide liquide par aération au moyen d'un fluide gazeux dans un rapport entre le fluide liquide et le fluide gazeux prédéfini, et la troisième étape de diminution consiste à ajuster le rapport entre le fluide liquide et le fluide gazeux en fonction de la concentration du au moins un composé organique mesuré dans le fluide du réseau.

Selon l'invention, le procédé industriel est un procédé de forage mettant en oeuvre un fluide de forage, et la troisième étape de diminution consiste à adapter la densité du fluide de forage en fonction de la concentration du au moins un composé organique mesuré dans le fluide du réseau.

Selon un autre mode de réalisation hors du cadre de l'invention, le procédé industriel est un procédé de désinfection d'eau par ajout d'au moins un réactif oxydant et la troisième étape de diminution consiste à adapter la quantité d'au moins un réactif (tel que chlore) ajouté en fonction de la concentration d'au moins un composé organique mesuré dans le fluide du réseau.

Selon un autre mode de réalisation hors du cadre de l'invention, le procédé industriel est un procédé de traitement d'eau potable, l'installation industrielle comprenant au moins un filtre avec media adsorbant en grain tel que charbon actif, ou une injection de media en poudre, tel que le charbon actif, et la troisième étape de diminution comprend une étape de variation du débit du fluide à travers un contacteur adsorbant / fluide , tel que filtre avec matériau adsorbant en grain, ou séparateur de media adsorbant en poudre /fluide. Le dosage du media adsorbant poudre peut également être adapté à l'abattement voulu du Ides composés organiques et au suivi en ligne.

Avantageusement, la troisième étape de diminution peut comprendre en outre une étape de régénération du media filtrant et la régénération du media se fait à fréquence fixe ou variable.

Hors du cadre de l'invention, le procédé industriel de traitement peut également être un procédé à base de membranes hydrophiles perméables aux fluides liquides ou à base de membranes hydrophobes perméables aux fluides gazeux, de type nano filtration ou osmose inverse.

L'invention concerne aussi un dispositif de contrôle de la concentration de composés organiques dans un fluide d'un réseau de fluide d'une installation industrielle selon la revendication 3.

Avantageusement, l'unité de correction comprend un dispositif d'injection dans le réseau de fluide d'un produit ayant pour effet de diminuer la concentration dudit au moins un composé organique

Selon un autre mode de réalisation, le dispositif de contrôle comprend un dispositif de télétransmission adapté à télétransmettre la concentration de chacun des composés organiques de la pluralité de composés organiques mesurée par l'analyseur à un centre de contrôle.

L'invention sera mieux comprise et d'autres avantages apparaîtront à la lecture de la description détaillée d'un mode de réalisation donné à titre d'exemple, description illustrée par le dessin joint dans lequel :
- la figure 1 représente schématiquement les étapes d'un procédé de contrôle de la concentration de composés organiques dans un fluide d'un réseau selon l'invention,
- la figure 2 représente schématiquement un mode de réalisation d'un dispositif de contrôle de la concentration de composés organiques dans un fluide d'un réseau selon l'invention,
- la figure 3 représente schématiquement un autre mode de réalisation d'un dispositif de contrôle de la concentration de composés organiques dans un fluide d'un réseau selon l'invention.

Par souci de clarté, les mêmes éléments porteront les mêmes repères dans les différentes figures.

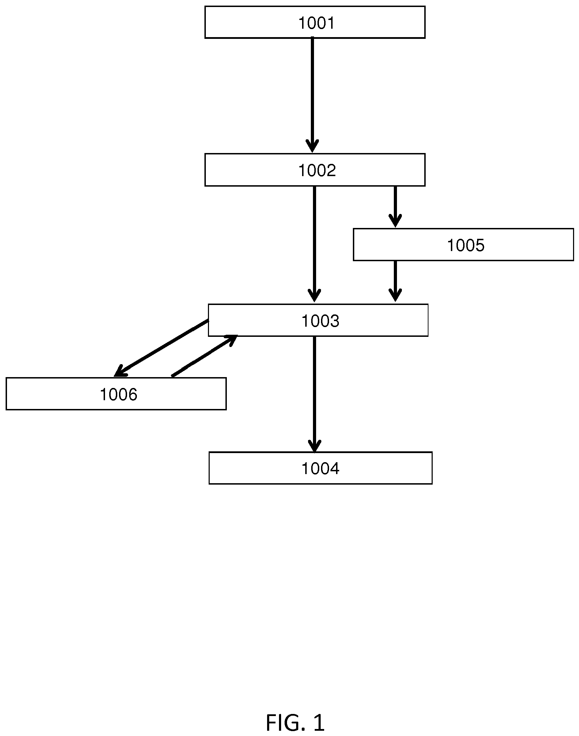

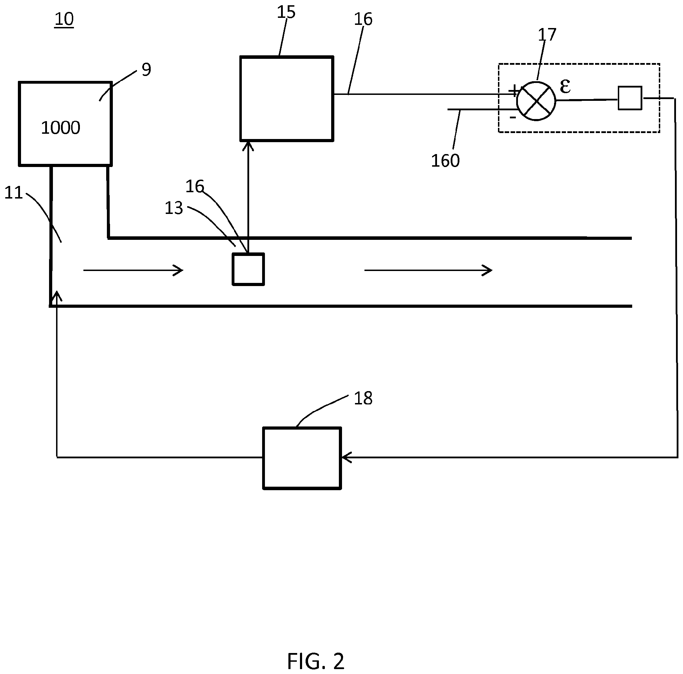

La **figure 1****,** en faisant aussi référence à la figure 2, représente schématiquement les étapes d'un procédé de contrôle de la concentration de composés organiques dans un fluide 11 d'un réseau selon l'invention. Le procédé de contrôle de la concentration de composés organiques dans le fluide 11 du réseau de fluide d'une installation industrielle, le fluide 11 comprenant une pluralité de composés organiques, comprend une première étape 1001 de mesure en ligne de la concentration 16 de chacun des composés organiques de la pluralité de composés organiques dans le fluide 11 du réseau de fluide par un analyseur 15, une deuxième étape 1002 de comparaison de la concentration 16 de chacun des composés organiques de la pluralité de composés organiques mesurée par l'analyseur 15 avec une valeur de seuil 160 prédéfinie pour chacun des composés organiques, comme valeur de consigne de traitement ou valeur maximale d'alarme, et une troisième étape 1003 de diminution de la concentration 16 d'au moins un composé organique si la concentration 16 dudit au moins un composé organique mesurée par l'analyseur 15 est supérieure à la valeur de seuil 160 prédéfinie correspondante.

L'analyseur 15 est configuré pour mesurer en ligne la concentration 16 de chacun des composés organiques de la pluralité de composés organiques présents dans le fluide 11. Il en résulte une pluralité de concentrations 16, une pour chaque composé organique mesuré. Et pour chacun des composés organiques, une valeur de seuil 160 est prédéfinie, chacune des valeurs pouvant être différente selon les normes en vigueur. Chaque concentration de composé organique mesurée est comparée à la valeur de seuil correspondant au composé organique dont il est question. Si la concentration de composé organique mesurée est supérieure à cette valeur de seuil, il faut une action correctrice correspondant à la troisième étape 1003 pour diminuer la concentration de ce composé organique.

La troisième étape 1003 de diminution peut notamment comprendre une étape 1004 d'injection dans le réseau de fluide 11 d'un produit ayant pour effet de diminuer la concentration du au moins un composé organique. L'injection du produit peut faire l'objet d'un asservissement contrôlé par la différence entre la concentration mesurée et la valeur de seuil prédéfinie de la concentration du composé organique.

Le procédé de contrôle selon l'invention peut comprendre préalablement à la troisième étape 1003 de diminution une étape 1005 de télétransmission de la concentration 16 de chacun des composés organiques de la pluralité de composés organiques mesurée par l'analyseur 15 à un centre de contrôle de l'installation de traitement. Le centre de contrôle peut alors également agir sur la concentration des composés organiques, éventuellement par une intervention humaine.

Un aspect particulièrement intéressant hors du cadre de l'invention s'applique à une installation industrielle 9 destinée à réaliser un procédé industriel 1000. Selon l'invention, la troisième étape 1003 de diminution consiste à agir sur au moins un paramètre 12 du procédé industriel 1000 ayant pour effet de diminuer la concentration 16 dudit au moins un composé organique ayant une concentration mesurée par l'analyseur 15 supérieure à sa valeur de seuil 160 prédéfinie.

Cet aspect permet donc, à partir de la mesure en ligne de la concentration de composés organiques, de piloter et d'optimiser (en termes de performance et coûts de fonctionnement) des procédés industriels dans le monde de l'eau et de l'environnement, par exemple, en contribuant à la prévention de la pollution industrielle par des solvants chlorés dans les ressources en eaux pour améliorer la filière de traitement. En pilotant des paramètres opérationnels sur l'installation industrielle en amont, l'aspect permet de contrôler des sous-produits de la désinfection par le chlore, tels que les trihalométhanes qui sont des composés organiques volatils, et qui font l'objet de recommandations par l'Organisation Mondiale de la Santé ou de valeurs limites aux Etats-Unis, en Europe et en Australie. Hors du cadre de l'invention, il est aussi possible d'identifier et traiter des pollutions dans l'air produites par exemple par la filière d'eaux usées et le traitement des boues qui génèrent des nuisances olfactives.

Selon un mode de réalisation hors du cadre de l'invention, le procédé industriel 1000 est un procédé de traitement d'un fluide liquide par aération au moyen d'un fluide gazeux dans un rapport entre le fluide liquide et le fluide gazeux prédéfini, et la troisième étape 1003 de diminution consiste à ajuster le rapport entre le fluide liquide et le fluide gazeux en fonction de la concentration du au moins un composé organique mesuré dans le fluide du réseau. Il en résulte un bon suivi de l'efficacité du procédé de traitement, aussi appelé procédé de stripping, et d'en optimiser les coûts d'exploitation.

Selon l'invention, le procédé industriel est un procédé de forage mettant en oeuvre un fluide de forage, et la troisième étape 1003 de diminution consiste à adapter la densité du fluide de forage en fonction de la concentration du au moins un composé organique mesuré dans le fluide du réseau. En d'autres termes, l'invention permet un bon suivi de forages contaminés avec pilotage du mélange des forages contaminés/non contaminés, de façon à respecter la législation sur l'eau distribuée.

Selon un autre mode de réalisation hors du cadre de l'invention, le procédé industriel est un procédé de désinfection d'eau par ajout d'au moins un réactif, et la troisième étape 1003 de diminution consiste à adapter la quantité du au moins un réactif ajouté en fonction de la concentration du au moins un composé organique mesuré dans le fluide du réseau.

Selon un autre mode de réalisation hors du cadre de l'invention, le procédé industriel est un procédé de traitement d'eau potable, l'installation industrielle comprenant au moins un filtre avec media adsorbant en grain, tel que le charbon actif en grain, ou bien une injection de media adsorbant en poudre ou un contacteur avec media adsorbant en poudre charbon actif ou un procédé de séparation sur membranes, et la troisième étape 1003 de diminution comprend une étape de variation du débit du fluide à travers le contacteur adsorbant/fluide, tel que le filtre avec matériau ou media adsorbant en grain, ou séparateur de media adsorbant en poudre/fluide ou dans le contacteur avec media adsorbant en poudre. La troisième étape 1003 peut aussi comprendre une étape de variation du dosage du media adsorbant en poudre. C'est-à-dire que le dosage du media adsorbant en poudre peut également être adapté à l'abattement voulu du/des composés organiques et au suivi en ligne.

Autrement dit, un filtre est situé au niveau de l'usine de traitement. Une usine de traitement d'eau potable comprend souvent des filtres à charbon actif destinés à éliminer des micropolluants. En jouant sur le débit de l'eau au travers de tels filtres et sur leur fréquence de régénération on peut améliorer l'élimination des composés organiques. Au niveau de l'usine, peut également être mis en oeuvre, un réacteur de contact eau/media adsorbant en poudre, tel que charbon actif en poudre, destinés à éliminer des micropolluants. En jouant sur le débit de l'eau au travers d'un tel contacteur et sur le dosage du média poudre, on peut améliorer l'élimination des composés organiques.

Avantageusement, la troisième étape 1003 de diminution comprend en outre une étape 1006 de régénération du media filtrant et la régénération du media se fait à fréquence fixe ou variable.

Hors du cadre de l'invention, le procédé industriel 1000 peut également être un procédé de traitement d'eau potable, l'installation industrielle comprenant au moins une membrane hydrophile perméable aux fluides liquides ou au moins une membrane hydrophobe perméable aux fluides gazeux, de type nano filtration ou osmose inverse. Et dans ce cas, la troisième étape 1003 de diminution comprend une étape de variation du débit du fluide à travers la membrane.

Suite à la mesure faite par l'analyseur 15, en cas de dépassement de la valeur réglementaire, le résultat de la mesure faite en ligne par l'analyseur 15 est utilisé pour rétroagir sur le fonctionnement de l'installation industrielle de traitement en abaissant par exemple la dose de chlore ou en optimisant l'élimination des matières organiques en agissant sur le taux de coagulant ou la dose de charbon actif en poudre, ou sur la fréquence de régénération des filtres à charbon.

La mise en oeuvre du procédé selon l'invention permet d'avoir une mesure de la concentration de composés organiques dans un fluide pertinente, car réalisée en ligne et en continu ou semi-continu, et un résultat utile pour améliorer le procédé industriel associé au fluide.

Le pilotage du procédé industriel se fait en temps réel, ce qui permet d'être plus réactif face à toute éventuelle pollution industrielle. Ce pilotage en temps réel n'est pas envisageable dans le cas de prélèvement de fluide pour analyse des concentrations des composés organiques en laboratoire, car le temps de retour est trop long. De plus, les composés organiques pouvant être des composés organiques volatils, avec une analyse en laboratoire, il y a un problème de précision de la mesure puisque le composé organique volatil a de fortes chances de ne plus être dans l'échantillon analysé. De ce fait, le pilotage du procédé industriel selon l'invention permet d'être plus pertinent et plus proche de la réalité en captant réellement la pollution et en la traitant de façon adaptée. L'avantage qui en découle est un gain de temps, de coût et de qualité du traitement du fluide.

La **figure 2** représente schématiquement un mode de réalisation d'un dispositif de contrôle10 de la concentration 16 de composés organiques dans un fluide 11 d'un réseau selon l'invention. Le dispositif de contrôle 10 de la concentration 16 de composés organiques dans le fluide 11 du réseau de fluide 11 d'une installation industrielle 9 adaptée à réaliser un procédé industriel 1000, comprend un analyseur 15 positionné à une première localisation 13 dans le réseau de fluide, adapté à mesurer en ligne la concentration 16 de chacun des composés organiques de la pluralité de composés organiques dans le fluide 11 à la première localisation 13. L'analyseur 15 est un appareil capable de mesurer en ligne, en continu ou semi-continu plusieurs composés organiques réglementés ou fréquemment retrouvés dans les eaux ou tout autre fluide concerné. Le dispositif de contrôle 10 comprend un comparateur 17 adapté à comparer la concentration 16 de chacun des composés organiques de la pluralité de composés organiques mesurée par l'analyseur 15 avec une valeur de seuil 160 prédéfinie pour chacun des composés organiques, et une unité de correction 18 adaptée à diminuer la concentration 16 d'au moins un composé organique dans le fluide 11, si la concentration dudit au moins un composé organique de la pluralité de composés organiques mesurée par l'analyseur 15 est supérieure à la valeur de seuil 160 prédéfinie correspondante.

L'analyseur 15 peut être par exemple un chromatographe en phase gazeuse portatif couplé à un micro-détecteur à ionisation d'argon, comprenant un dispositif de prélèvement. L'analyseur 15 peut comprendre deux sondes, l'une de température et l'autre permettant l'arrivée d'argon dans le fluide. Deux types de récipients peuvent y être fixés. L'un est utilisé pour faire les tests en statique et peut être jaugé à 2L. Le second récipient sert aux analyses en ligne. Il est muni de deux ouvertures, l'une pour l'arrivée du fluide et l'autre pour la sortie, connectées à des tuyaux pour former le circuit. Avantageusement, l'analyseur 15 se met en route dès son branchement à une prise électrique. Le traitement de données est réalisé sur un ordinateur équipé d'un logiciel adapté et relié en réseau avec l'analyseur 15. Il doit être connecté à une bouteille d'argon pour fonctionner. L'analyseur en ligne est fait pour fonctionner en continu avec une maintenance minimale. L'analyse peut être conduite sans prétraitement ou filtration préalable pour des eaux peu chargées.

Pour les analyses d'eau, cette dernière circule en continu dans une cellule inférieure. A une fréquence programmable, les composés organiques de l'eau circulante sont strippés par un gaz neutre (en l'occurrence l'argon), entraînés par l'argon sur un adsorbant qui les piège. Ce dernier est ensuite désorbé thermiquement et les composés organiques sont entraînés par le gaz vecteur argon sur une colonne de chromatographie qui les sépare physiquement. Les composés séparés sont ensuite détectés au moyen d'un détecteur à microionisation d'argon nécessitant l'argon pour son fonctionnement. Ce système fonctionne donc sur le principe d'espace de tête dynamique.

Il est à noter qu'il s'agit d'un exemple d'analyseur 15 pouvant être mis en oeuvre dans le cadre de l'invention. L'invention peut être appliquée avec tout type d'analyseur capable de séparer les composés organiques individuellement en ligne en continu ou semi-continu afin d'en déterminer la concentration.

Avantageusement, l'unité de correction 18 peut comprendre un dispositif d'injection dans le réseau de fluide 11 d'un produit ayant pour effet de diminuer la concentration dudit au moins un composé organique

Selon un autre mode de réalisation, le dispositif de contrôle 10 comprend un dispositif de télétransmission adapté à télétransmettre la concentration 16 de chacun des composés organiques de la pluralité de composés organiques mesurée par l'analyseur à un centre de contrôle.

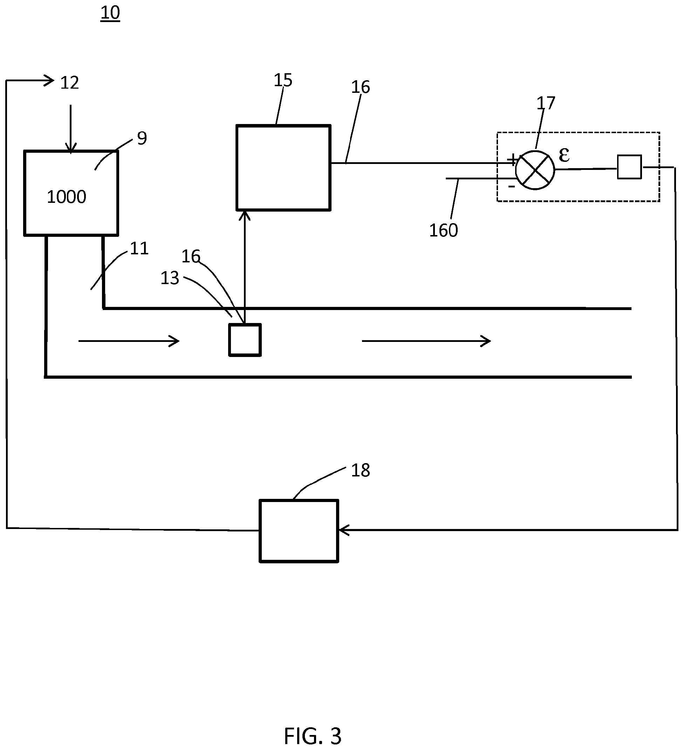

La **figure 3** représente schématiquement un autre mode de réalisation du dispositif de contrôle 10 de la concentration de composés organiques dans le fluide 11 du réseau selon l'invention. Le procédé industriel 1000 fait intervenir au moins un paramètre 12, l'unité de correction 18 comprend un premier module de pilotage configuré pour agir sur le au moins un paramètre 12 du procédé industriel 1000 ayant pour effet de diminuer la concentration dudit au moins un composé organique en fonction de l'évolution de la concentration dudit au moins un composé organique mesuré.

Un tel dispositif de contrôle 10 selon l'invention permet donc d'obtenir une mesure en ligne de la concentration de composés organiques dans un fluide, et de piloter le procédé industriel associé au fluide. Plus précisément, un tel dispositif de contrôle permet d'identifier et traiter des pollutions en agissant sur un paramètre de fonctionnement du procédé industriel afin d'adapter au mieux le fonctionnement de l'installation industrielle tout en prenant en compte le niveau des composés organiques qui en résultent. Comme déjà mentionné, les composés organiques peuvent être des solvants chlorés dans l'eau, des trihalométhanes, mais aussi des additifs notamment dédiés aux carburants comme l'éther éthyle tertio butyle ou ETBE (pour l'acronyme anglo-saxon Ethyl Tert-Butyl Ether), ou l'éther méthyle tertio butyle ou MTBE (pour l'acronyme anglo-saxon Methyl Tert-Butyl Ether), ou bien même des métabolites d'algues odorants sous réserve que le seuil de détection de l'analyseur soit suffisamment bas. Bien entendu, l'invention s'applique à tout composé organique, volatil.

## Revendications

1. Procédé de contrôle de la concentration de composés organiques volatils dans un fluide (11) d'un réseau de fluide d'une installation industrielle (9), l'installation (9) étant destinée à réaliser un procédé industriel (1000), le fluide (11) comprenant une pluralité de composés organiques volatils, comprenant les étapes suivantes :
• première étape (1001) de mesure en ligne de la concentration (16) de chacun des composés organiques volatils de la pluralité de composés organiques volatils dans le fluide (11) du réseau de fluide par un analyseur (15),
• deuxième étape (1002) de comparaison de la concentration (16) de chacun des composés organiques volatils de la pluralité de composés organiques mesurée par l'analyseur (15) avec une valeur de seuil (160) prédéfinie pour chacun des composés organiques volatils, comme valeur de consigne de traitement ou valeur maximale d'alarme,
• troisième étape (1003) de diminution de la concentration d'au moins un composé organique volatil si la concentration (16) dudit au moins un composé organique volatil mesurée par l'analyseur (15) est supérieure à la valeur de seuil (160) prédéfinie correspondante,
le procédé industriel (1000) étant un procédé de forage mettant en oeuvre un fluide de forage, le procédé étant **caractérisé en ce que** le fluide de forage comprend un mélange fluide contaminé/non contaminé et la troisième étape (1003) de diminution consiste à adapter la densité du fluide de forage en fonction de la concentration du au moins un composé organique volatil mesuré dans le fluide du réseau par le pilotage du mélange fluide contaminé/non contaminé.

2. Procédé de contrôle selon la revendication 1, **caractérisé en ce qu'**il comprend préalablement à la troisième étape (1003) de diminution une étape (1005) de télétransmission de la concentration (16) de chacun des composés organiques volatils de la pluralité de composés organiques volatils mesurée par l'analyseur (15) à un centre de contrôle.

3. Dispositif de contrôle (10) de la concentration de composés organiques volatils dans un fluide (11) d'un réseau de fluide d'une installation industrielle (9) adaptée à réaliser un procédé industriel (1000), le fluide (11) comprenant une pluralité de composés organiques volatils, le dispositif de contrôle (10) comprenant :
• un analyseur (15) positionné à une première localisation (13) dans le réseau de fluide,
l'analyseur (15) étant adapté à mesurer en ligne la concentration (16) de chacun des composés organiques volatils de la pluralité de composés organiques volatils dans le fluide (11) à la première localisation (13),
• un comparateur (17) adapté à comparer la concentration (16) de chacun des composés organiques volatils de la pluralité de composés organiques volatils mesurée par l'analyseur (15) avec une valeur de seuil (160) prédéfinie pour chacun des composés organiques volatils,
• une unité de correction (18) adaptée à diminuer la concentration (16) d'au moins un composé organique volatil dans le fluide (11), si la concentration (16) dudit au moins un composé organique volatil de la pluralité de composés organiques mesurée par l'analyseur (15) est supérieure à la valeur de seuil (160) prédéfinie correspondante,
ledit dispositif de contrôle (10) étant **caractérisé en ce que** le procédé industriel (1000) est un procédé de forage mettant en oeuvre un fluide de forage, le fluide de forage comprenant un mélange fluide contaminé/non contaminé, et l'unité de correction (18) comprend un premier module de pilotage configuré pour adapter la densité du fluide de forage en fonction de la concentration du au moins un composé organique volatil mesuré dans le fluide du réseau par le pilotage du mélange fluide contaminé/non contaminé.

4. Dispositif de contrôle (10) selon la revendication 3, **caractérisé en ce qu'**il comprend un dispositif de télétransmission adapté à télétransmettre la concentration (16) de chacun des composés organiques volatils de la pluralité de composés organiques volatils mesurée par l'analyseur (15) à un centre de contrôle.

## Patentansprüche

1. Verfahren zur Überwachung der Konzentration flüchtiger organischer Verbindungen in einem Fluid (11) eines Fluidnetzes einer Industrieanlage (9), wobei die Anlage (9) dazu bestimmt ist, einen industriellen Prozess (1000) durchzuführen, das Fluid (11) umfassend eine Vielzahl von flüchtigen organischen Verbindungen, umfassend die folgenden Schritte:
· einen ersten Schritt (1001) eines Inline-Messens der Konzentration (16) von jeder der Vielzahl von flüchtigen organischen Verbindungen in dem Fluid (11) des Fluidnetzes durch ein Analysegerät (15),
· einen zweiten Schritt (1002) eines Vergleichens der von dem Analysegerät (15) gemessenen Konzentration (16) von jeder der Vielzahl von flüchtigen organischen Verbindungen mit einem Schwellenwert (160), der für jede der flüchtigen organischen Verbindungen als Behandlungsvorgabewert oder maximaler Alarmwert vordefiniert ist,
· einen dritten Schritt (1003) eines Verringerns der Konzentration von mindestens einer flüchtigen organischen Verbindung, wenn die von dem Analysegerät (15) gemessene Konzentration (16) der mindestens einen flüchtigen organischen Verbindung höher ist als der entsprechende vordefinierte Schwellenwert (160),
wobei das industrielle Verfahren (1000) ein Bohrverfahren unter Verwendung eines Bohrfluids ist, wobei das Verfahren **dadurch gekennzeichnet ist, dass** das Bohrfluid ein Gemisch aus kontaminiertem/nicht kontaminiertem Fluid umfasst und der dritte Schritt (1003) eines Verringerns darin besteht, die Dichte des Bohrfluids abhängig von der Konzentration der mindestens einen flüchtigen organischen Verbindung, die in dem Fluid des Netzwerks gemessen wird, durch die Steuerung des Gemischs aus kontaminiertem/nicht kontaminiertem Fluid anzupassen.

2. Steuerungsverfahren nach Anspruch 1, **dadurch gekennzeichnet, dass** es vor dem dritten Schritt (1003) eines Verringerns einen Schritt (1005) einer Fernübertragung der Konzentration (16) von jeder der Vielzahl von flüchtigen organischen Verbindungen, die von dem Analysegerät (15) gemessen wird, an eine Steuerzentrale.

3. Steuerungsvorrichtung (10) der Konzentration flüchtiger organischer Verbindungen in einem Fluid (11) eines Fluidnetzes einer Industrieanlage (9), die angepasst ist, um einen industriellen Prozess (1000) durchzuführen, wobei das Fluid (11) eine Vielzahl flüchtiger organischer Verbindungen umfasst, die Steuerungsvorrichtung (10) umfassend:
· ein Analysegerät (15), das an einer ersten Stelle (13) in dem Fluidnetz positioniert ist,
wobei das Analysegerät (15) angepasst ist, um die Konzentration (16) von jeder der Vielzahl von flüchtigen organischen Verbindungen in dem Fluid (11) an der ersten Stelle (13) Inline zu messen,
· einen Komparator (17), der angepasst ist, um die von dem Analysegerät (15) gemessene Konzentration (16) von jeder der Vielzahl von flüchtigen organischen Verbindungen mit einem vordefinierten Schwellenwert (160) für jede der flüchtigen organischen Verbindungen zu vergleichen,
· eine Korrektureinheit (18), die angepasst ist, um die Konzentration (16) von mindestens einer flüchtigen organischen Verbindung in dem Fluid (11) zu verringern, wenn die Konzentration (16) der mindestens einen flüchtigen organischen Verbindung aus der Vielzahl von organischen Verbindungen, die von dem Analysator (15) gemessen wird, größer ist als der entsprechende vordefinierte Schwellenwert (160),
wobei die Steuerungsvorrichtung (10) **dadurch gekennzeichnet ist, dass** der industrielle Prozess (1000) ein Bohrverfahren unter Verwendung eines Bohrfluids ist, wobei das Bohrfluid ein Gemisch aus kontaminiertem/nicht kontaminiertem Fluid umfasst, und die Korrektureinheit (18) ein erstes Ansteuerungsmodul umfasst, das konfiguriert ist, um die Dichte des Bohrfluids abhängig von der Konzentration der mindestens einen flüchtigen organischen Verbindung, die in dem Fluid des Netzes gemessen wird, durch Ansteuern des kontaminierten/nicht kontaminierten Fluidgemischs anzupassen.

4. Steuerungsvorrichtung (10) nach Anspruch 3, **dadurch gekennzeichnet, dass** sie eine Fernübertragungsvorrichtung umfasst, die angepasst ist, um die Konzentration (16) von jeder der Vielzahl von flüchtigen organischen Verbindungen, die von dem Analysator (15) gemessen wird, an eine Steuerzentrale zu übertragen.

## Claims

1. A method for controlling the concentration of volatile organic compounds in a fluid (11) of a fluid network of an industrial plant (9), the plant (9) being intended to carry out an industrial method (1000), the fluid (11) comprising a plurality of volatile organic compounds, comprising the following steps:
· first step (1001) of on-line measurement of the concentration (16) of each of the volatile organic compounds of the plurality of volatile organic compounds in the fluid (11) of the fluid network by an analyser (15),
· second step (1002) of comparing the concentration (16) of each of the volatile organic compounds of the plurality of organic compounds measured by the analyser (15) with a threshold value (160) predefined for each of the volatile organic compounds, as a treatment setpoint value or maximum alarm value,
· third step (1003) of reducing the concentration of at least one volatile organic compound if the concentration (16) of said at least one volatile organic compound measured by the analyser (15) is greater than the corresponding predefined threshold value (160),
the industrial method (1000) being a drilling method using a drilling fluid, the method being **characterised in that** the drilling fluid comprises a contaminated/non-contaminated fluid mixture and the third step (1003) of reduction consists in adapting the density of the drilling fluid as a function of the concentration of the at least one volatile organic compound measured in the network fluid by controlling the contaminated/non-contaminated fluid mixture.

2. Control method according to claim 1, **characterised in that** it comprises, prior to the third step (1003) of reduction, a step (1005) of remote transmission of the concentration (16) of each of the volatile organic compounds of the plurality of volatile organic compounds measured by the analyser (15) to a control centre.

3. A device (10) for monitoring the concentration of volatile organic compounds in a fluid (11) of a fluid network of an industrial plant (9) adapted to carry out an industrial method (1000), the fluid (11) comprising a plurality of volatile organic compounds, the monitoring device (10) comprising:
• an analyser (15) positioned at a first location (13) in the fluid network, the analyser (15) being adapted to measure on-line the concentration (16) of each of the plurality of volatile organic compounds in the fluid (11) at the first location (13),
• a comparator (17) adapted to compare the concentration (16) of each of the volatile organic compounds of the plurality of volatile organic compounds measured by the analyser (15) with a predefined threshold value (160) for each of the volatile organic compounds,
• a correction unit (18) adapted to decrease the concentration (16) of at least one volatile organic compound in the fluid (11), if the concentration (16) of said at least one volatile organic compound of the plurality of organic compounds measured by the analyser (15) is greater than the corresponding predefined threshold value (160),
said monitoring device (10) being **characterised in that** the industrial method (1000) is a drilling method using a drilling fluid, the drilling fluid comprising a contaminated/uncontaminated fluid mixture, and the correction unit (18) comprises a first control module configured to adapt the density of the drilling fluid as a function of the concentration of the at least one volatile organic compound measured in the network fluid by controlling the contaminated/non-contaminated fluid mixture.

4. A monitoring device (10) as claimed in claim 3, **characterised in that** it comprises a remote transmission device adapted to transmit the concentration (16) remotely of each of the volatile organic compounds of the plurality of volatile organic compounds measured by the analyser (15) to a monitoring centre.
